Europäisches Patentamt

European Patent Office

Office européen des brevets

(11) Veröffentlichungsnummer: **0 227 929**
**B1**

(12) **EUROPÄISCHE PATENTSCHRIFT**

(45) Veröffentlichungstag der Patentschrift: **25.04.90**

(51) Int. Cl.⁵: **A 61 B 17/22**

(21) Anmeldenummer: **86115672.7**

(22) Anmeldetag: **12.11.86**

(54) **Wasserkissen zur Verwendung bei der berührungsfreien Lithotripsie.**

(30) Priorität: **18.12.85 DE 3544707**

(43) Veröffentlichungstag der Anmeldung:
**08.07.87 Patentblatt 87/28**

(45) Bekanntmachung des Hinweises auf die
Patenterteilung:
**25.04.90 Patentblatt 90/17**

(84) Benannte Vertragsstaaten:
**FR GB IT**

(56) Entgegenhaltungen:
**DE-A-2 925 933**
**DE-A-3 503 702**

(73) Patentinhaber: **DORNIER MEDIZINTECHNIK GMBH**
**Postfach 1128**
**D-8034 Germering 1 (DE)**

(72) Erfinder: **Wess, Othmar, Dr.**
**Max-Nadler-Strasse 17**
**D-8000 München 81 (DE)**
Erfinder: **Grözinger, Reiner**
**Greppenstrasse 9**
**D-8031 Alling (DE)**
Erfinder: **Windsheimer, Manfred**
**Ludwigstrasse 2**
**D-8034 Germering (DE)**
Erfinder: **Isdebski, Kai**
**Bigenweilerstrasse 27**
**D-7968 Saulgau (DE)**

(74) Vertreter: **Landsmann, Ralf, Dipl.-Ing.**
**DORNIER GMBH - Patentabteilung - Kleeweg 3**
**D-7990 Friedrichshafen 1 (DE)**

EP 0 227 929 B1

## Beschreibung

In Klasse A 61 B 17/22 der IPC sind Vorrichtungen enthalten, die in der berührungsfreien Lithotripsie mittels Stosswellen Verwendung finden. Ein technisches Problem ist dabei, die Einkoppelung der Stosswelle in den menschlichen Körper mittels eines Koppelmediums. Am besten bewährt hat sich hierzu die Lagerung der Patienten in einer wassergefüllten Wanne, in der sich auch die Stosswellenquelle befindet.

Es ist schon vorgeschlagen worden, die Verbindung zum Patienten im Bereich des Konkrements mittels einer Membran, die die Stosswellenquelle abschliesst oder mittels eines Wasserkissens, das sich zwischen Stosswellenquelle und der Haut des Patienten befindet, herzustellen.

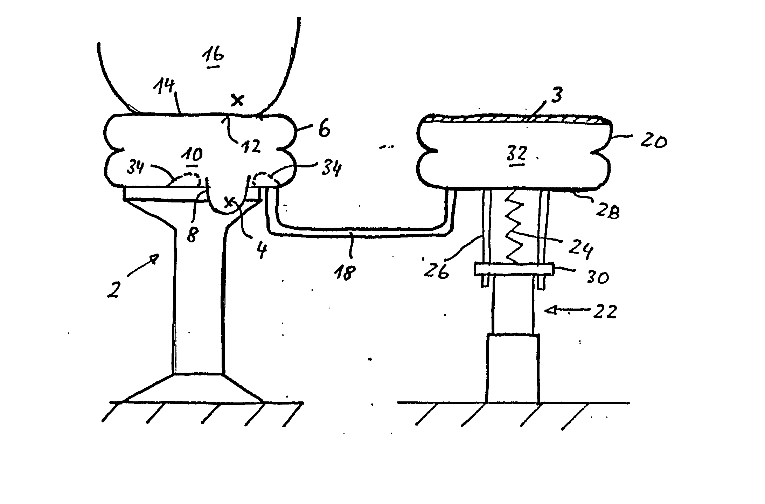

Aus der DE—A—2,925,933 (Beschreibung zu Fig. 4 auf Seite 20) ist eine fluidgekoppelte Ultraschall-Sondenanordnung bekannt. Dabei umfasst der Kopplungsabschnitt einen Zylinder, der an einem Ende mit einem Kopplungsbeutel und am anderen Ende mit einer Leitung verbunden ist. Der Kompensationsabschnitt umfasst einen Zylinder mit gleitbar eingesetztem Kolben, an dem ein Gewicht mit vorbestimmter Masse angebracht ist. In dieser Ausführung enthalten der Kopplungsbeutel, die Zylinder und die Leitung jeweils ein fluides Kopplungsmittel, z. B. Wasser.

Der Erfindung liegt die Aufgabe zugrunde, eine Ankoppeleinrichtung für die wannenfreie Lithotripsie zu schaffen, die ein Wasserkissen aufweist, dessen Niveau einstellbar ist und beim Eindrücken des Wasserkissens durch einen Körper der Anpressdruck des Wasserkissens an diesen Körper konstant gehalten wird, so dass vor und während der Behandlung der Abstand zwischen der Stosswellenquelle und dem Konkrement konstant bleibt.

Diese Aufgabe wird erfindungsgemäss dadurch gelöst, dass das Kissen mittels einer Schlauchleitung mit einem zweiten Kissen verbunden ist, das als Druckausgleichskissen auf einem höhenverstellbaren Tisch mittels Druckfedern mit bestimmter Dimensionierung und Federkennlinie gelagert ist.

Bei einer vorteilhaften Ausgestaltung der Erfindung befindet sich am oberen Ende des Druckausgleichskissens eine gewichtserhöhende Platte aus Metall oder Kunststoff.

Die Erfindung wird nachfolgend anhand einer Figur näher erläutert.

Die einzige Figur zeigt dier erfindungsgemässe Vorrichtung in prinzipieller Darstellung.

An einem Behandlungstisch 2 mit integrierter Stosswellenquelle 4 befindet sich ein als Faltenbalg gestaltetes Kissen 6, das mit Wasser gefüllt ist. Der Reflektor 8 der Stosswellenquelle 4 ragt in den wassergefüllten Raum 10 des Kissens 6. Am anderen Ende des Kissens befindet sich eine Membran 12, die luftspaltlos an der Haut 14 eines zu behandelnden Patienten 16 anliegt. Der Patient 16 befindet sich auf einer nicht gezeigten Patientenliege und die in seinem Körper zu behandelnde Stelle ist mit x gekennzeichnet.

Vom Kissen 6 führt eine Schlauchleitung 18 zu einem zweiten Kissen 20, das ebenfalls als Faltenbalg gestaltet ist. Dieses Druckausgleichskissen 20 befindet sich auf einem Hubtisch 22, der mechanisch, elektrisch oder hydraulisch in der Höhe verstellbar ist. Zwischen dem Hubtisch 22 und dem zweiten Kissen 20 sind eine oder mehrere Spiralfedern 24 angeordnet und es sind Führungsstangen 26 vorhanden, die einerseits an einer Grundplatte 28 des Kissens 20 befestigt sind und andererseits in einer Platte 30 des Hubtischs 22 geführt werden.

Im Raum 32 des Kissens 20 befindet sich ebenfalls Wasser. Das Kissen kann an seiner Oberseite mit einer gewichtserhöhenden Platte 3 abgeschlossen sein.

Funktion.

Der Patient 16 wird mittels einer nicht gezeigten Patientenliege in die Behandlungsposition gebracht. Der Hubtisch 22 wird nach oben gefahren, so dass das zweite Kissen 20 höher zu liegen kommt als das Kissen 6 und Wasser über die Schlauchleitung 18 zum Kissen 6 strömt. Die Membran 12 des Kissens 6 geht nach oben und es erfolgt die Ankopplung an die Haut 14 des Patienten 16. Werden nun die gestrichelt dargestellten Balge 34 für eine Röntgenortung zur exakten Positionierung des Patienten ausgefahren, so kann das dadurch verdrängte Wasser über die Schlauchleitung 18 zum zweiten Kissen 20 abfliessen und bewirkt hier, dass sich die Feder 24 um einen bestimmten Federweg, der sich aus der Federkennlinie ergibt, absenkt. Der Druck im Kissen 6 wird aufgrund der Dimensionierung der Feder 24 konstant gehalten und ändert sich nicht, wenn der Patient exakt positioniert wird und wenn die Balge 34 wieder eingefahren werden, worauf die Behandlung des Patienten beginnen kann.

Würde man zur Konstanthaltung des Drucks hier ein System aus Pumpen, Ventilen und Sensoren verwenden, so ergäbe sich ein Regelkreis, der permanent schwingt, so dass die erfindungsgemässe passive Regelung vorzuziehen ist.

## Patentansprüche

1. Wassergefülltes Kissen für die berührungsfreie Lithotripsie, das flächenhaft und luftspaltlos an die Haut eines Patienten im Bereich des zu behandelnden Konkrements angelegt wird, dadurch gekennzeichnet, dass das Kissen (6) mittels einer Schlauchleitung (18) mit einem zweiten Kissen (20) verbunden ist, das als Druckausgleichskissen auf einem höhenverstellbaren Tisch (22) mittels Druckfedern (24) mit bestimmter Dimensionierung und Federkennlinie gelagert ist.

2. Vorrichtung nach Anspruch 1, dadurch gekennzeichnet, dass sich am oberen Ende des Druckausgleichskissens (20) eine gewichtserhöhende Platte (3) aus Metall oder Kunststoff befindet.

**Revendications**

1. Coussin rempli d'eau pour la lithotritie sans contact qui est appliqué à plat et sans passage d'air contre la peau d'un patient dans la région de la concrétion à traiter, caractérisé en ce que le coussin (6) est relié au moyen d'un tuyau flexible (18) à un second coussin (20) qui, en tant que coussin de compensation de pression, est monté sur une table (22) réglable en hauteur au moyen de ressorts de pression (24) présentant des dimensions et une courbe caractéristique de ressort déterminées.

2. Dispositif selon la revendication 1, caractérisé en ce qu'à l'extrémité supérieure du coussin de compensation de pression (20) est disposée une plaque (3) en métal ou en matière plastique pour augmenter le poids.

**Claims**

1. Water-filled cushion for non-contact lithotripsy which is placed against the skin of a patient in an areal manner without any air gaps, in the region of the concretion which is to be treated, characterised in that the cushion (6) is connected by means of a hose line (18) to a second cushion (20) which is mounted as a pressure-equalising cushion on a vertically adjustable table (22) by means of pressure springs (24) with particular dimensions and spring characteristics.

2. Apparatus as claimed in claim 1, characterised in that a weight-increasing plate (3) of metal or plastic material is located at the upper end of the pressure-equalising cushion (20).